# EUROPEAN PATENT APPLICATION

(11) **EP 3 101 420 A1**
(43) Date of publication of application: **07.12.2016**
(21) Application number: 15170744.5
(22) Date of filing: 04.06.2015
(51) Int. Cl.: G01N 33/02, G01N 22/00, G01N 21/3586

(54) **SENSOR FOR MONITORING FREEZING STATUS OF PRODUCTS**

(71) Applicant: M2Wave bvba, 1040 Brussel (BE); VRIJE UNIVERSITEIT BRUSSEL, 1050 Brussel (BE)
(72) Inventor: STIENS, Johan, 2820 Bonheiden (BE); VANDERMEIREN, Werner, 1930 Zaventem (BE); PANDEY, Gokarna, 1200 Woluwe-Saint-Lambert (BE); DIMICCOLI, Luca, 1180 Ukkel (BE)
(74) Representative: DenK iP

(57) **Abstract**

A sensor system for sensing a level of frozenness of a predetermined product is described. The sensor system comprises at least one emission antenna for directing electromagnetic radiation with at least one frequency within a frequency range of 0.1 GHz to 10 THz to a product and at least one receiver for receiving said electromagnetic radiation after transmission through or reflection from said product. The sensor system furthermore comprises at least one data processing unit for determining a level of frozenness based on said received electromagnetic radiation and taking into account dielectric properties of the predetermined product based on a physical-mathematical model of the product or calibration data.

## Description

### Field of the invention

The present invention relates to the field of freezing and thawing of products. More particularly the present invention relates to sensor devices and methods for measuring a level of frozenness of a product, e.g. food products, in a non-destructive and contact-free manner.

### Background of the invention

One of the most important methods of preservation includes freezing. Perishable products or products whose properties need to be kept inalterable for long periods of time are usually frozen and kept at low temperatures, under the melting point of water, for a desired time. This is normally done with food and agricultural produce, organs for transplantation, biological and bioengineering materials, medicines and pharmacological products, and chemical products in general. Another method of preservation is lyophilization, which normally includes two steps of dissecation and one step of freezing. This method is usually applied to foodstuffs (vegetables, beverages, etc), pharma (vaccines, blood, etc) and biotechnological and chemical materials (proteins, enzymes, artificial skin, etc), and occasionally to ceramic products (e.g. in semiconductor industry) or restoration (in archeology and recovery of water-damaged documents or goods).

The freezing process causes several state changes in the products. These are controlled by measuring variables such as the temperature or pressure. For example, in food industry, the freezing lines are equipped with numerous sensors, such as pressure, humidity and temperature sensors, for controlling the freezing process in-line. However, these measurements can only give an idea about the level of frozenness of the product under consideration. This usually gives a large uncertainty about the actual state of the product at the end of the freezing line. In ordinary productions, to ensure that the product is law-regulation compliant, the product throughput and the freezer settings are based on optimization of the process, obtained from gained experience, typicaly through destructive tests, and a calculated risk. In case the product is packed near the freezing line, an additional test on the product can be gained from inserting, on a random basis, a dedicated temperature probe into a package. This measurement method is indirect, intrusive, labor-intensive, simplistic and it usually can only provide information of the state of the product surface, or the environment in proximity of the product, which leaves uncertainty about the state of the product at its kernel, as the outside layers may be completely frozen but the interior may be still unfrozen.

Methods such as producing indentations on the product and visual inspection have similar shortcomings. Additionally, none of these methods take into account the heterogeneity of morphology (e.g. the humidity and gradient of humidity, the ice fraction, etc) of the products at a macro scale.

Besides, these methods can only be applied in food industry.

In case of lyophilisation industry the two drying steps can be well-monitored by various methods but not the freezing step. As freezing is usually the first step, it is the most critical, because when this is not well executed, the consecutive steps will be influenced.

Hence, it is still necessary to optimize the freezing process and its monitorization in terms of throughput and energy consumption, and also applicable to a wide range of industries.

### Summary of the invention

It is an object of embodiments of the present invention to provide good methods and systems for providing reliable measure of the degree of frozenness of a product under consideration.

It is an advantage of particular embodiments of the present invention to provide the means to translate data collected by the sensor into the degree of frozenness of the product or other physical properties of the product of interest.

It is an advantage of particular embodiments of the present invention that time dependent monitoring of parameters, such as for example temperature, watercontent, ice content, etc. can be determined allowing measuring different stages of a freezing or thawing process. It thus is an advantage of embodiments that a freezing or thawing process can be monitored and optimized.

It is an advantage of embodiments of the present invention to provide methods and systems for optimizing industrial freezing processes in terms of efficiency and product throughput.

It is an advantage of embodiments of the present invention to provide a mobile apparatus for quality control of frozen products in cold stores.

It is an advantage of embodiments according to the present invention that a full longitudinal cross-section (with respect to the sensor field-of-view) of the product is sensed for determining the degree of frozenness of the product and not only the product surface or the environment in proximity of the product, thus allowing to provide better information regarding the level of frozenness or the freezing or thawing process.

It is also an advantage of particular embodiments of the present invention, that a high transverse sensing resolution can be achieved e.g. by limiting the transverse cross-sectional field-of-view of the sensor and/or the exposed area of the product.

It is also an advantage of particular embodiments of the present invention, that the degree of frozenness can be determined in a fast way, allowing for example to determine a dregree of frozenness for free-falling product.

It is also an advantage of particular embodiments of the present invention, that the degree of frozenness during the freezing/thawing process can be determined by combining measurements from multiple sensors, each measuring products with a different freezer residence time. In other words, according to at least some embodiments a plurality of sensors can be used for detecting differenent states of a freezing/thawing process.

The data acquisition may be triggered when the product is in proximity of the sensor. It is also an advantage of particular embodiments of the present invention that the sensor inpinges radiation on the product only when necessary, allowing automatization of the process and saving energy.

It is an advantage of embodiments of the present invention that a quantification of the freezing status for various types of products (compositions, size, thickness, etc) can be obtained by measuring the S-parameters with GHz-THz electromagnetic waves.

It is an advantage of embodiments accordin to the present invention that a fast and cheap detection can be obtained since in some embodiments, the radiation frequency used for monitoring may be limited to a single frequency or frequency band. It is an advantage that using a single frequency or frequency band still can allow to obtain sufficient information to provide an accurate level of frozenness. In some embodiments of the present invention the radiation also may be limited to a single polarization and a single angle irradiation, providing further speed and cost advantages. It thereby is an advantage that product and process specific knowledge can be used. Being able to limit the number of irradiation parameters, e.g. to limit to a single irradiation frequency and/or a single angle or polarization of the probing signal is especially advantages as at least some applications are based on high throughput of materials.

It is an advantage of embodiments of the present invention that only a single measurement is required for obtaining accurate information regarding the level of frozenness. S-parameters can be obtained through a transmission measurement only, a reflective measurement only or a combination of transmission and reflection measurements. It is an advantage of at least some embodiments that the level of frozenness can be obtained using only a single transmission measurement. The latter allows to obtaine a low cost system for detecting a level of frozenness or for monitoring a freezing or thawing process.

It is an advantage of embodiments of the present invention that accurate results can be obtained. The system may be adapted such that the sensor is configured with respect to the product holder in such a way that the distance with respect to the product to be measured is small. In lens-less solutions the distance may be selected between a distance corresponding with the beginning of the far field of the antenna and a distance such that the filling factor of the product in the field of view of the sensor is above a set threshold value. The beginning of the far field of the antenna may depend on the ratio of the antenna size and the wavelength and may sometimes be expressed as 2xDxD/wavelength. In a lens-based solution the distance may for example be such that it is at substantially the focal distance of the lens system with an allowed variation (away from the focal distance) in the distance such that the filling factor of the product in the field of view is still above a set threshold value. In this way, a good interaction strength between the radiation and the product can be ascertained since the emitter antenna and the product are close to each other.

The data processing unit may be programmed for taking into account the product thickness of the predetermined product for determining the fraction of frozen water of the freezable water content present in the predetermined product.

Where in embodiments according to the present invention reference is made to the freezable water content, reference is made to all water that can be frozen, independent whether it is in frozen condition or liquid condition. Freezable water content does not include for example water that cannot be frozen, e.g. water that is in a bound state.

At least one of the emitter antenna or the receiver are separated from a region where the predetermined product is present, through a protective window. The protective window may be dimensioned to have anti-reflectif properties or wherein the protective window comprises a dedicated anti-reflective coating.

According to at least some embodiments of the present invention, the system may include a fill-factor detection in order to guarantee a sufficient interaction strength between the radiation and the product. According to some embodiments, the sensing system may have a field-of-view or irradiation area that is being matched with the shape and/or size of the products.

According to at least some embodiments of the present invention, the sensing system may comprise a protective window shielding the emitter antenna from the products. Such a protective window may for example have anti-reflective (AR) features. It is an advantage of at least some embodiments according to the present invention that the sensing system can be provided in an industrial compatible way, i.e. allowing for example water clearning, industrial environmental conditions, etc.

It is an advantage of embodiments of the present invention that a specific level of frozenness can be determined based on the measured S parameter(s) by making use of product related information. In some embodiments, use may be made of a mapping function generated by a physic-mathematical model and/or calibration experiments.

It is an advantage of embodiments of the present invention that since the freezing and/or thawing process can be monitored and evaluated, this also allows controlling the freezing and/or thawing process by activally optimizing it through various control parameters of a freezing and/or thawing system, such as for example temperature, wind speed, timing of the thawing of condensors, food load, throughput controly such as throughput speed, etc. These sensors thus also allow to optimize the food process (e.g. freezing) in terms of product throughput and energy consumption with real-time expert knowledge about the degree of frozenness for the product under consideration.

Other features and advantages of embodiments of the present invention will become apparent from the ensuing description, taken in conjunction with the accompanying drawings.

The present invention relates to a sensor system for sensing a level of frozenness of a predetermined product, the sensor system comprising
- at least one emission antenna for directing electromagnetic radiation with at least one frequency within a frequency range of 0.1 GHz to 10 THz to the product,
- at least one receiver for receiving said electromagnetic radiation after transmission through or reflection from said product,
- at least one data processing unit for determining a level of frozenness based on said received electromagnetic radiation and taking into account dielectric properties of the predetermined product based on a physical-mathematical model of the product or calibration data. The receiver and the emission antenna may be configured such that the receiver is positioned along the propagation direction of the electromagnetic radiation emitted by the emission antenna.

The electromagnetic radiation may be electromagnetic radiation of a single frequency or single frequency band. The use of a single frequency or single frequency band allows reducing the cost and complexity of the sensor system, compared to the use of a spectrometric system. Nevertheless, the system does not need to be limited to a single frequency or single frequency band system.

The at least one emission antenna and the at least one receiver may be configured with respect to each other and a product holder for holding the product so that the sensor system operates in transmission mode.

The data processing unit may be adapted for taking into account a physical-mathematical model expressing a freezable water content being present in the predetermined product. It is an advantage of embodiments of the present invention that the change of the freezable water content during the freezing or thawing process can be accurately measured using radiation within a frequency range of 0.1 GHz to 10 THz, resulting in the possibility for accurately detecting the level of frozenness.

The physical-mathematical model may be a parameterized physical-mathematical model.

The emission antenna and the receiver may form a single transceiver.

At least one of the emitter antenna or the receiver may be separated from a region where the predetermined product is present, through a protective window. The protective window may comprise an anti-reflective coating.

The sensor system furthermore may comprise a product transporting means, wherein the emission antenna, the receiver and the product transporting means are configured for detecting a level of frozenness of the predetermined product at a particular position of the predetermined product on the product transporting means. The system may comprise a plurality of receivers along the product transporting means for detecting the level of frozenness at different positions along the product transporting means. The sensor system may comprise a modulator for modulating the impinging radiation. The sensor may present better signal-to-noise ratio. The sensor system may comprise a further receiver for allowing to measure both transmission and reflection.

The sensor system may comprise allowing to monitor a freezing or thawing process. The sensor system may be embedded or partly embedded in a closes system. Such a closed system may at least partly have Teflon based walls.

The system may comprise a fill factor detector for detecting a fill factor.

The frozenness monitoring system may be adapted for accepting or rejecting a measurement when the fill factor is sufficiently high and/or wherein the data processing unit is adapted for taking into account the fill factor when determining a level of frozenness. The sensor system may comprise a detection trigger for activating the monitoring system upon detection of a product in a predetermined position with respect to the sensor. The sensor system may comprise a radiation focusing means. The sensor system may comprise an oscillator for sweeping the frequency of the electromagnetic radiation impinging on the predetermined product. The sensor may comprise a thickness measurement means for determining a thickness of the predetermined product.

The present invention also relates to a method of detecting a level of frozeness of a predetermined product, the method comprising the steps of
- emitting electromagnetic radiation towards the predetermined product, the electromagnetic radiation having at least one frequency within a frequency range of 0.1 GHz to 10 THz - detecting electromagnetic radiation transmitted through or reflected from the predetermined product, and
- calculating a level of frozenness of the predetermined product based on said received electromagnetic radiation and taking into account dielectric properties of the predetermined product based on a physical-mathematical model of the product or calibration data. The method furthermore may comprise analyzing the freezing or thawing process based on said level of frozenness. The method furthermore may evaluating a quality of a freezing or thawing process that was implied earlier to a predetermined product. The latter can be derived based on the occurrence of a memory effect present in the S-parameters.

The level of frozenness of the predetermined product is calculated based on said received electromagnetic radiation and taking into account a dielectric properties of the predetermined product based on a physical-mathematical model of the product or calibration data and a thickness of the predetermined product.

The level of frozenness of the predetermined product is calculated based on said receiving electromagnetic radiation and taking into account a dielectric properties of the predetermined product based on a physical-mathematical model of the product or calibration data and a fill factor.

The present invention also relates to a computer program product programmed for receiving data regarding an amount of electromagnetic radiation with at least one frequency within a frequency range of 0.1 GHz to 10 THz directed to the product and regarding an amount of electromagnetic radiation received after transmission through or reflection from said product, the computer program product further being programmed for determining a level of frozenness based on said received electromagnetic radiation and taking into account dielectric properties of the predetermined product based on a physical-mathematical model of the product or calibration data. The computer program product may be adapted for performing, when executed on a computing device, a method according to an embodiment of the present invention.

The present invention furthermore relates to a freezing or thawing system, the system comprising a sensor system as described above, whereby the system furthermore is adapted with a controller for controlling the freezing or thawing of products taking into account a determined level of frozenness. The freezing or thawing system may comprise heaters or coolers and the controller may be adapted for controlling the heaters and/or the coolers depending on the determined level of frozenness.

The present invention also relates to the use of a sensor system as described above, for controlling a freezing or thawing system, or for analyzing a freezing or thawing cycle that was implemented on a predetermined product.

Particular and preferred aspects of the invention are set out in the accompanying independent and dependent claims. Features from the dependent claims may be combined with features of the independent claims and with features of other dependent claims as appropriate and not merely as explicitly set out in the claims.

These and other aspects of the invention will be apparent from and elucidated with reference to the embodiment(s) described hereinafter.

### Brief description of the drawings

FIG. 1 illustrates two schematic representations of GHz-THz based sensors comprising an electromagnetic transmitting apparatus and the receiving apparatus on the left, and a transceiver and receiver apparatus on different sides of a product carrier, according to an embodiment of the present invention.
FIG. 2 illustrates two configurations of the sensor for a flux of products, in free fall on the left, and in glide/slide-and-fall configuration on the right, according to an embodiment of the present invention.
FIG. 3 illustrates a configuration of the apparatus positioned above a reflective product-carrying medium, according to an embodiment of the present invention. On the left, the transmitter and receiver can be alligned for maximization of radiation detection. On the right, a transceiver is located on the product side of a reflective product-carrying medium.
FIG. 4 illustrates a view of a product under test, showing the the full longitudinal cross-sectional product-volume according to the sensors field-of-view which may be sensed, according to an embodiment of the present invention.
FIG. 5 illustrates further configurations of embodiments of the present invention comprising EM lenses and/or an aperture means (reflective or absorptive), for limiting the exposed area of the product.
FIG. 6 illustrates further configurations of embodiments of the present invention, comprising an aperture for limiting the exposed area of the product (left) and further comprising a fill factor detector (right).
FIG. 7 illustrates a configuration of the sensor comprising a transmitter and receiver, a product-carrying medium and means for measuring product thickness, according to an embodiment of the present invention.
Fig. 8 illustrates a receiver apparatus featuring a special surface or EM lens (left image) or a reflective or absoptive surface (right image), for focusing or limiting the incoming electromagnetic waves onto a predetermined area of the receiver, as can be used in an embodiment of the present invention.
FIG. 9 illustrates a receiver apparatus featuring an aperture for limiting the incoming electromagnetic waves onto a predetermined area of the receiver, as can be used in an embodiment of the present invention.
FIG. 10 illustrates a frozeness monitoring system comprising a product-carrying medium, plurality of sensors, and a data processing unit, according to an embodiment of the rpesent invention.
FIG. 11 illustrates an exemplary sensor output of two sensors in a frozeness monitoring system for an exemplary full freezing cycle of a freezing line, according to an embodiment of the present invention.
FIG.12 illustrates the relative sensors filling factor given by the relative portion of the sensors cross-sectional field-of-view covered by the food product at a given time instance.

The drawings are only schematic and are non-limiting. In the drawings, the size of some of the elements may be exaggerated and not drawn on scale for illustrative purposes.

Any reference signs in the claims shall not be construed as limiting the scope.

In the different drawings, the same reference signs refer to the same or analogous elements.

### Detailed description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practiced without these specific details. In other instances, well-known methods, structures and techniques have not been shown in detail in order not to obscure an understanding of this description.

The present invention describes sensor devices and measurement methods for measuring or monitoring, qualitatively or quantitatively, the degree of frozenness of a product in a reliable and contact-free manner. The degree of frozenness thereby may allow to study freezing or thawing processes. The degree or level of frozenness (LOF), for various products, independently of their composition, thickness or morphology, can be defined as the ratio of the fraction of a predetermined substance in a solid phase and the total amount of the substance present, in a given volume. In embodiments of the present invention, the LOF of substances whose phase changes upon freezing or melting (e.g. water) can be measured. In some embodiments of the present invention suitable for measurement of products containing water molecules, the LOF is calculated by obtaining the ratio of ice and the total amount of freezable water present in the sensed volume, e.g. in the cross-sectional volume-field-of-view of the sensor.

Water molecules, whose dielectric properties are modeled by the Debye-Lorentz model, are strongly influenced by their phase state and temperature. The interaction of GHz - THz waves, used in embodiments of the present invention, with a product is dependent on the dielectric properties of that product. When freezing a product comprising dipolar molecules, the dielectric property might change dramatically compared with the dielectric property of the same product at room temperature, due to temperature and phase state changes in the product. Such changes can be detected using transmission and/or reflection measurements using waves within a frequency range of 0.1 GHz to 10 THz.

Whereas in embodiments of the present invention reference is often given to freezing, it will be clear to the person skilled in the art that systems and methods also encompass controlling and/or determining a degree of frozenness during thawing, drying or evaporating.

Where in embodiments of the present invention reference is made to GHz to THz waves, the electromagnetic emission might consist of one or more frequencies or a frequency band or one or more frequency bands, e.g. chosen to achieve the desired penetration depth for sensing the core of the product, as this region will typically take the longest to freeze. This allows to perform a longitudinal volume measurement of the product under consideration. Where reference is made to GHz - THz frequencies reference is made to frequencies within the range 0.1 GHz to 10 THz.

Where in embodiments of the present invention reference is made to "S-parameter", reference is made to parameters obtained by obtaining measurements comprising the reflection (R), the transmission (T), or both R and T, of radiation within a frequency range of 0.1 GHz to 10 THz. These S-parameters are inherently determined by the dielectric property distribution throught the volume of the product. Embodiments of the present invention make use of the fact that during freezing or thawing of a product, the dielectric property and/or dielectric property distribution is not constant, and may also be dependent on parameters such as the temperature. In certain embodiments, dielectric property also depends of the amount of water, the amount of water in ice form, etc. which may substantially change with time. The present invention is not limited thereto, and it may be used to monitorize any other suitable parameters, and changes thereof, related to the dielectric property and/or dielectric property distribution for freezing or thawing.

In a first aspect, embodiments of the present invention provide a sensor system for sensing the level of frozenness of a product. The sensor system according to embodiments of the present invention comprises at least one emission antenna for directing electromagnetic radiation with at least one frequency within a frequency range of 0.1 GHz to 10 THz to the product. The sensor system also comprises at least one receiver for receiving said electromagnetic radiation after transmission through or reflection from said product. Furthermore, the sensor system comprises at least one data processing unit for determining a level of frozenness based on the received electromagnetic radiation and taking into account a physic-mathematical model related to the dielectric property of the predetermined product. The frequency of the electromagnetic radiation may be varied within a range, for example by a voltage-controlled oscillator, which may be connected to the transmitter to sweep the emitted wavelength within a certain range. In other embodiments, the frequency of radiation is fixed.

The system can operate both in transmission and in refelction. In some embodiments of the present invention, the emission antenna may emit radiation within a frequency range of 0.1 GHz to 10 THz to a product, and the receiver may receive transmitted radiation, transmitted through the product. In such embodiments, the parameter S comprises the transmission parameter. In embodiments of the present invention, more generally, the parameter S may comprise reflection parameters, transmission parameters or a combination of transmission and reflection parameters. In further embodiments, the parameter S may comprise both transmission and reflection parameters by adding a surface which reflects the signal transmitted through the product and bounces it back to the transceiver.

The present invention may further take into account a number of parameters relating to the impinging radiation, for example the frequency and polarization of the radiation, the angle of incidence, etc. In some embodiments of the present invention, the radiation may be limited to a single frequency, single polarization, and single angle of incidence. The present invention advantageously allows taking into account the distance between the emission antenna of the transmitter or transceiver and the product, as well as shape, size and thickiness and relate these factors with the field-of-view, through a fill-factor. The fill factor may be defined as the power fraction of the emitted intensity distribution which is intercepted by the product area. The emitted intensity distribution thereby refers to the intensity distribution that is seen by the receiver in absence of any products. For example, one can emit a certain intensity distribution (e.g. a Gaussian beam, or other beam-shape). Even without any products in between the emitter and receiver we will capture only a part of this intensity distribution with our receiving antenna. This part is our "reference intensity distribution". When there is a product in between the emitter and receiver, partially intercepting the "reference intensity distribution", the filling factor then is the power ratio of the integral of that part of the reference intensity distribution over the intercepted area define by the product and the power contained in our reference intensity distribution. The fill factor therefore is considered from the side of view of the receiver. 100% filling factor would mean that all power received at the receiver was interacting with the product, which does not necessary means that all power emitted from the emitter interacted with the product!

Embodiments of the present invention may be advantageously readily applied in any industry, e.g. it may be compatible with water cleaning, industrial, pharmaceutical or (bio-)hazarous environmental conditions. In such cases, the emitting and receiving antennas may be installed in suitable enclosures. One example of enclosure material that may be used is Teflon. Each enclosure may comprise a window that is transparent for the considered waves. The enclosures may comprise any suitable sealings and protective windows with anti-reflective features, for example a predetermined thickness in order to achieve anti-reflective properties for the emission wavelength and/or spectrum used, although the present invention is not limited thereby. The window of the tranmitter or tranceiver may be designed to focus the electromagnetic radiation onto a predetermined and/or adjustable area of the product, and the window of the receiver may be designed to foculs electromagnetic power on the receiver, e.g. the receiving antenna. The enclosure may further comprise a cooling means and/or a means to put the enclosure under pressure.

In embodiments of the present invention, a data processing unit is enclosed as indicated above. According to embodiments of the present invention, the data processing unit may determine the level of frozenness based on the obtained measurements and based on a physic-mathematical model of the product. Such a model can be a simple model expressing e.g. only a content of a certain component, e.g. water, in the product. For other products, more complicated models may be used. Another example of a model that can be used is a food product comprising e.g. the following components: water, fat, carbohydrates, proteins. The sum of the relative fractions of all components is equal to 100%. The fraction of water is divided into non-freezable water and freezable water. The latter one can be in the liquid or in the solid state (=ice). Each of the components is characterized by its temperature and phase state dependent dielectric properties. For each of the materials, temperature and phase state dependent properties are supposed to be known: e.g. heat capacity, conductivity, permittivity. By introducing a dielectric mixing formula, known to specialists in the field, one can derive the dynamic dielectric properties of the food product as a whole. By extent a product can be more divided into more constituting elements if needed.

For measuring, different configurations may be used. For example, the system may have batch processing, storage configuration, continuous processing, or any other configuration. The system may even combine configurations, such as combining continuous processing with storage configuration.

In batch processing, the product or products are not in motion. The LOF and any variation of LOF (due to freezing or thawing) can be determined by a sensor or set of sensors, which may be static or movable. Storage configuration is equivalent to multiple batches for products with different history. The measurement may be performed N times, the data processed and may be averaged.

In continuous processing, a product-carrying medium also referred to as a product transporting means, is added to the system, such as a conveyor belt, a lineshaft roller conveyor, vertical conveyors, spiral conveyors, electric tracks or any other suitable method for bringing one or more products into the measurement range of a sensor or set of sensors, in a "processing line" configuration. For example, a conveyor belt combined with a free fall of the product may be advantageously used.

Additionally, the data acquisition may be triggered when the product is in proximity of the sensor. It is also an advantage of particular embodiments of the present invention that the sensor inpingues radiation on the product only when necessary, allowing automatization of the process and saving energy.

Different configurations may be applied with great flexibility to continuous processing, depending on each particular application. The following configurations are exemplary embodiments and do not limit the present invention.
- In case of products with similar or identical thickness, texture and/or composition, the product may be larger or smaller than the area exposed to the radiation beam:
   a) In the first case (product larger than exposed area), the radiation would interact with part of the product. The measurement may be performed by "sample and hold" and data acquisition of the S parameters a number N of times.
      A decision algorithm may determine the rejection or adoption of sensor data for further processing. This may be included in a high-throughput line, for quantification of the LOF of various products, independently of their variation of thickness, morphology, composition, etc.
      When accepted, the measurements are sent to a data processing unit where they may be further processed (e.g. average, median, max, min...) and extract the result, which is converted via a mapping function into variables such as the dryness, maximum and minimum temperature through the volume of the measured region of the product, and the LOF. Several measurements may be performed on the same product, e.g. in different places of a product. An aperture or EM radiation focusing means may be used to limit the field of view, advantageously increasing the transverse sensing resolution. A full longitudinal cross section may be advantageously sensed, determining the LOF of the cross section, and not only of the surface.
   b) In the second case (product smaller than exposed area), limiting the radiation may be possible (e.g. by adding a suitable lens or an aperture means). Alternatively or additionally, the measurement may be performed when the beam size is filled above a given threshold filling factor (for example, by adding a filling factor detector and a decision algorithm). The rest of the procedure (sample and hold measurement, etc) may be the same as in the case a).
- In case of products with different thickness, further thickness measurements may be added to any of the cases a) or b). The thickness data may be independent data (e.g. when thickness is measured with a thickness sensor), and then is sent to the data processing unit, where is combined with the S-parameters measured. In case the thickness data is dependent data, several measurements of S-parameters may be performed in the same region of the product or products, in different stages of cooling or freezing (e.g. at different places along a processing line). Thickness independent data may be then extracted.
- In case of products with varying texture, composition and/or morphology, the S-parameter measured with the sensor (sample and hold measurement, etc) may be measured, optionally with thickness measurement. The average of N measurements of data points (such as S-parameters) is performed and sent to the data processing unit. The mapping function may obtain absolute LOF, or relative LOF, as well as the level of dryness and the temperature, independently of morphology differences.

The present invention allows combination of measurements from multiple sensors, and multiple types of sensors, which may measure products with a different freezer residence time. The relative signal difference between individual sensors may, after calibration, be used as a measure for the degree of frozenness of the product.

For example, one sensor may be used to obtain the LOF of a product and allow a decision algorithm to reject the product, allow it for further processing or return it to the freezing cycle. Two or more sensors in different stages of a freezing line may monitor the LOF and variations thereof.

A sensor system according to the first aspect of the present invention may be applied to freezing process in cold rooms or low temperature storage, and it may also be applied to mobile modules, for example portable fridges or trucks.

In a second aspect of the present invention, embodiments of the present invention comprise a method for calculating the LOF of a product, and optionally monitoring variations thereof. The method of detecting a level of frozeness of a predetermined product comprises emitting electromagnetic radiation towards the predetermined product, the electromagnetic radiation having at least one frequency within a frequency range of 0.1 GHz to 10 THz and detecting electromagnetic radiation transmitted through or reflected from the predetermined product. Based on these results as well as taking into account a physic-mathematical model related to the dielectric property of said predetermined product, a level of frozenness is determined.

Embodiments of the present invention may utilize a radiation of an intensity under a determined threshold over which the dielectric properties of the product are altered for determining whether something has frozen or thawn(e.g. polar molecules vibrate with a frequency such that thawing processes start). Further embodiments may comprise a calibration step, in which the total content of polar substances, e.g. water, is measured e.g. by other methods, or by the present method, e.g. the total content of water when the product is defrosted.

Embodiments of the present method may be carried out by a monitoring system according to embodiments of the first aspect of the present invention. The method may comprise other sub-steps, for example bringing a product into contact with a sensor or data analysis, which may be carried out in a computer.

In a third aspect of the present invention, embodiments of the present invention comprise computer methods for translating reflection and transmission data into the degree of frozenness of the product. Physical-mathematical models and/or calibration experiments may take into account factors such as the product geometry, its homogeneity and composition, the emission spectrum and the environmental conditions, as well as other parameters such as the product thickness and/or shape, which is advantageous for accurately determining the degree of frozenness of the product. If a physical-mathematical model is used, such model may be a parameterized model.

The freezing process under consideration can be fully modelled. Dedicated numerical 4-dimensional (x, y, z, time) heat-transfer models shall give as a result a 3 dimensional temperature and phase state distribution inside the product under test, taking into account the geometry, the composition and the environment surrounding the product. These results are then translated into a dynamic dielectric property distribution as a function of temperature, phase state, time and wavelength by making use of mixing formulas known in the prior art. The reflection and transmission coefficients or S-parameters may then be dynamically obtained by means of an electromagnetic forward solver. Based on these simulations, a mapping function can be built in order to translate reflection and / or transmission spectra into degree of frozenness values.

The algorithm may be adapted to products with different morphologies, sizes and compositions, according to each application; e.g. an algorithm adapted for vegetables, or for meats, may present different physical-mathematical model characteristics from an algorithm adapted for medicines and vaccines, and the calibration may be different as well.

It is an object of particular embodiments of the third aspect of the present invention to provide means for translating data collected by a sensor within a frequency range of 0.1 GHz to 10 THz (e.g. a sensor according to the first aspect of the present invention) into the LOF of the product.

In the following, particular exemplary embodiments are discussed with reference to the figures, nonetheless these embodiments are not limiting.

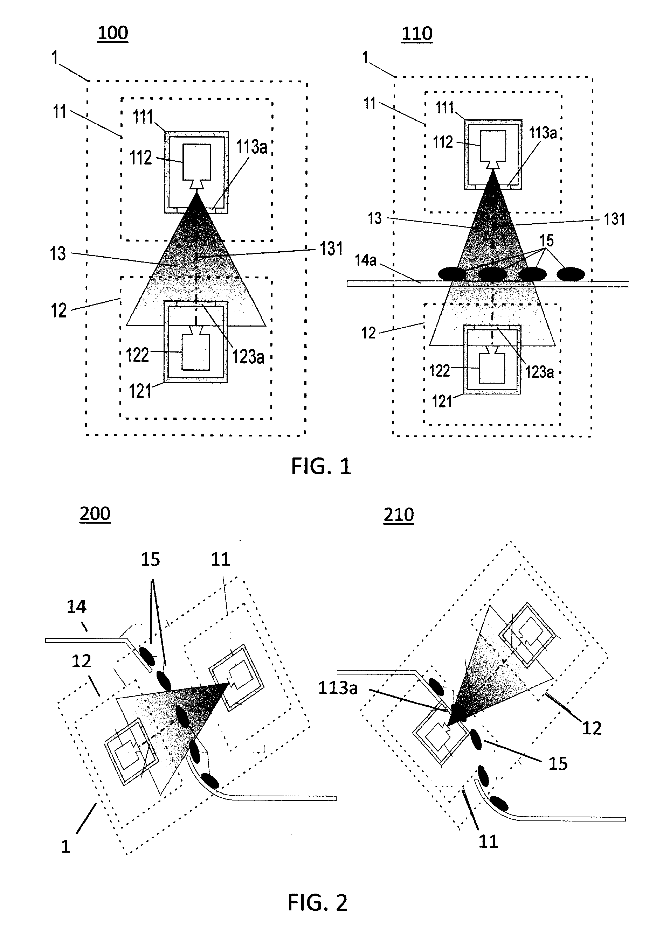

In FIG. 1, left scheme 100, a basic sensor 1 using radiation within a frequency range of 0.1 GHz to 10 THz is illustrated. It comprises a transmitter 11, which may be a transceiver, and a receiver 12. Typically the transmitter and receiver apparatus are both aligned along the propagation axis 131 in order to maximize the received electromagnetic power 13. The transceiver apparatus 11 is enclosed in an industrially graded enclosure 111 provided with a transparent window 113a, comprising of electromagnetic transmitting or transceiving means 112 (e.g. at least one antenna), and the receiving apparatus 12 enclosed in an industrially graded enclosure 121 provided with a transparent window 123a comprising of electromagnetic receiving means 122. The electromagnetic receiving means 122 may comprise for example an antenna, a low noise amplifier and a detector. The electromagnetic wave emission 13 may be a continuous wave or modulated. In case of modulated electromagnetic wave emission, the receiving means in the transceiver and/or receiver apparatus may be equipped with lock-in functionality for improving the signal-to-noise ratio of the received signals. It should be noted that the receiving functionality of the transceiver apparatus may or may not be implemented depending on the measurement needs.

The right scheme 110 of FIG. 1 shows a product under test 15 passing on a product carrying medium 14a (e.g. a conveyor belt) through the sensor cavity between the transmitter 11 and receiver 12. In this particular embodiment, the carrying medium may be substantially transparent for the emission wavelength range of the particular embodiment, depending on the sensor configuration which applies.

FIG. 2 show different possibilities for product carrying mediums. The left scheme 200 shows a carrying medium comprising a free fall of products 15. The products cross the sensor during the fall. A detector may optionally be used to trigger the sensor according to embodiments of the present invention when the product crosses the space between the transmitter and the receiver. The right scheme 210 of FIG. 2 shows a free fall after the product has been impinged with radiation from the transmitter or transceiver 11. For example, the products 15 may glide or slide down the aperture 113a of the transmitter or transceiver 11, the radiation is transmitted through the product 15 and then captured by the receiver.

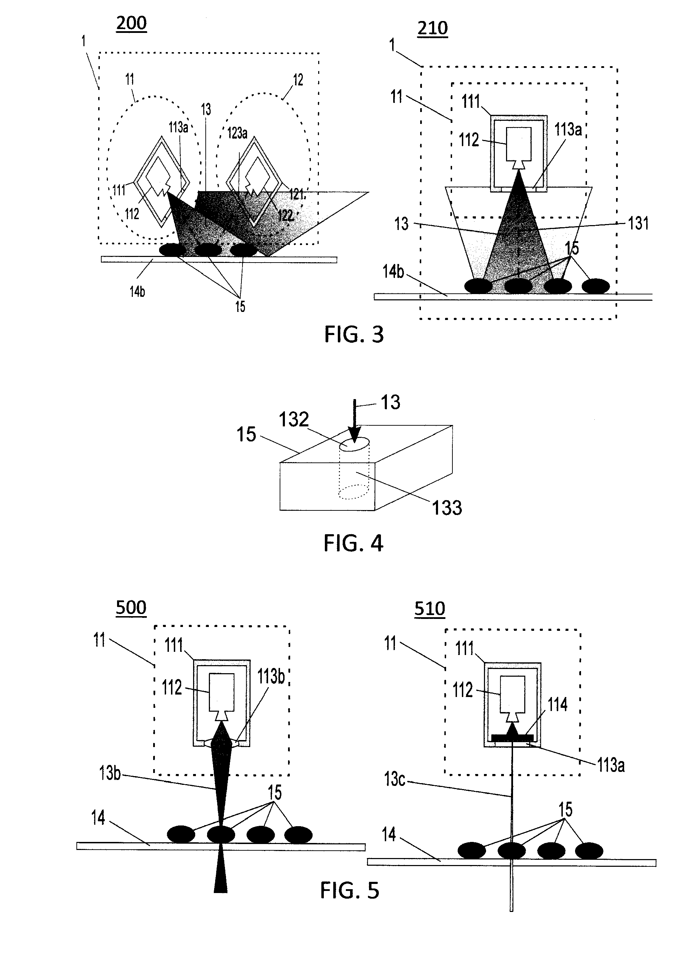

FIG. 3 shows different configurations of the sensor with respect to the radiation impinging direction. The left scheme 300 of FIG. 3 shows a configuration in which the transmitter or transceiver 11 and receiver 12 can be aligned forming an angle with the product. For example, transceiver 11 and receiver 12 can be rotated for a better control of the incidence and collection angle. The product carrying medium 14b is reflective, such that the emitted radiation can be captured after its interaction with the product 15 under inspection. The right scheme 310 of FIG. 3 also presents a reflective product carrying medium, and only a transceiver 111 is needed. The information collected by the transceiver may comprise reflected radiation and reflection transmitted through the product 15, reflected on the product carrying medium 14b and transmitted again through the product 15.

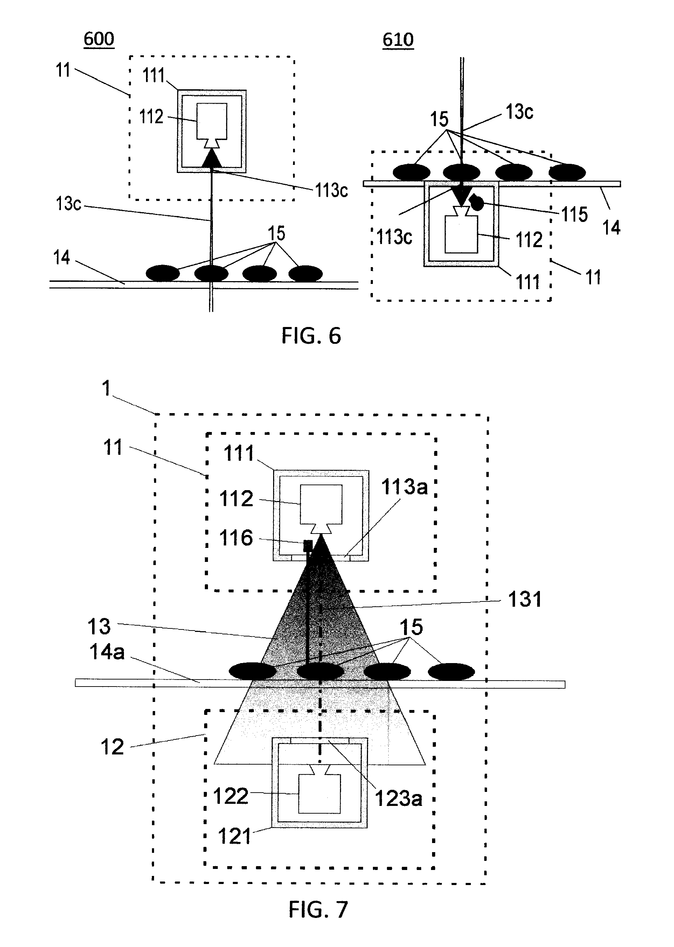

FIG. 1 to 3 show configurations for which the product is smaller than the area of incidence of radiation. A cross section of the product volume may be sensed, which is advantageous for studies of LOF distribution, for example in a heterogeneous product. FIG. 4 shows a perspective view of a product 15 under test, the full longitudinal cross-sectional product volume 133 according to the sensor field of view 132 of the electromagnetic power 13. This volume 133 is the amount of volume which may be sensed from 15. Several exemplary configurations utilizing a field of view 132 smaller than the total volume of the product 15 are shown in FIG. 5 and 6.

The left scheme 500 of FIG. 5 shows a transmitter or transceiver 11 equipped with focusing means 113b, which may be integrated in the transparent window, in order to focus the electromagnetic beam 13b to obtain a cross-sectional field-of-view 132 of the sensor 1 substantially equal or smaller than the product 15 itself. The transmitter/receiver configuration may be as in the scheme 110 of FIG. 1, comprising a further receptor (not pictured), or as in FIG. 3, comprising a transceiver and a reflective conveyor belt. The right scheme 510 of FIG. 5 shows a transmitter (or transceiver) 11 equipped with aperture means 114 in order to limit the electromagnetic beam 13c (rather than focus it); reducing the cross-sectional field-of-view 132 of the sensor 1 to an area which is substantially equal or smaller than the product 15 itself.

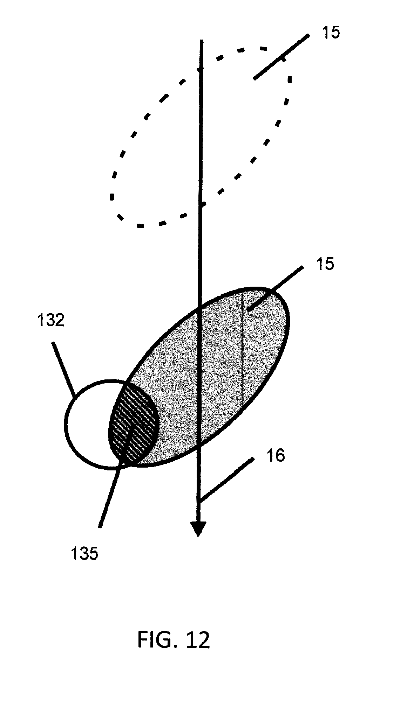

The left scheme 600 of FIG. 6 shows a transmitter or tranceiver 11 equipped with aperture means 113c integrated in the window, in order to limit the electromagnetic beam 13c as before. The right scheme 610 of FIG. 6 shows a transmitter or transceiver 11 equipped with a similar aperture means 113c, which can be integrated into the product carrying medium 14. It may further comprise a detector 115 for detecting the sensors aperture filling factor 135. It may be used to initiate the acquisition of measurement data. Example implementations of the sensors aperture filling factor detection means 115 may be a light detector or a distribution of light detectors, an impedance meter, an acoustic sensor, a laser-based sensor, a camera, etc., the present invention not being limited thereto. It may be a dedicated sensor using radiation within a frequency range of 0.1 GHz to 10 THz. Optionally, the transceiver 11 itself may be used as a sensors aperture filling factor detection means, in this case. The functionality of product detection means 115 may also be used to assess the portion of the transparent window covered by the flux of products. It is an advantage of embodiments according to the present invention that a substantially full cross-sectional volume 133, according to the sensors field-of-view 132, may be sensed for determining the LOF of the product 15. It is also an advantage of the present invention that the measurement of the relative filling factor of the sensors cross-sectional field-of-view with the passing product 15 can be used to interpret the quality / relevance of the measurement of that particular product under consideration. For example, in some embodiments of the present invention, the quality may enable the decision to accept or discard acquired reflection of transmission measurement at any moment in time, which may be automatized via an algorithm. The relative filling factor is illustrated in FIG. 12 and can be defined as the relative portion 135 of the cross-sectional field-of-view area 132 which is covered by the product 15. The relative filling factor will be dependent on time as the product follows its trajectory indicated by arrow 16.

FIG. 7 shows a sensor 1 and a substantially transparent product carrying means 14a, further equipped with a product thickness detection means 116. The product thickness may be used by the interpretation algorithm in order to deliver an output proportional to the LOF of the product and independent on its thickness. Some mplementations of the product thickness measurement means may be, for example, visible or IR laser distance meter based on trigonometry or on time-of-flight, visible or IR camera or line detector, acoustic distance sensor, etc.

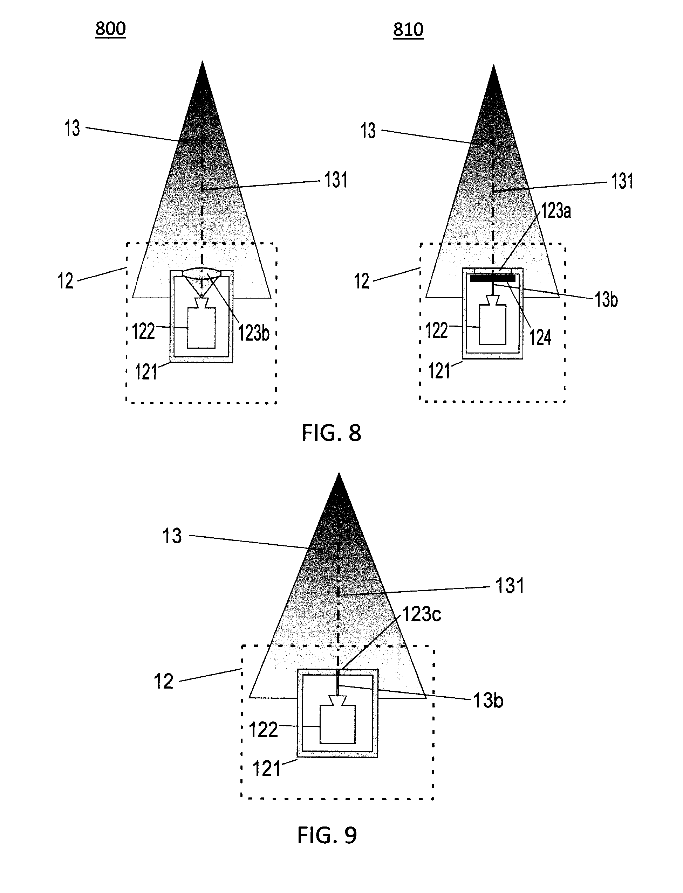

The left scheme 800 of FIG. 8 shows a receiver 12 equipped with focusing means, which may be optionally integrated in the transparent window 123b. The focusing means can substantially focus the electromagnetic beam 13 onto the receiving means 122 of the sensor. The right scheme 810 of FIG. 8 shows a receiver 12 equipped with aperture means 124 in the transparent window 123a, in order to limit the cross-sectional field-of-view 132 of the sensor to an area which is substantially equal or smaller than the product 15 itself. Integration of the aperture is optional. For example, FIG. 9 shows a receiver 12 with an aperture integrated in the window 123c.

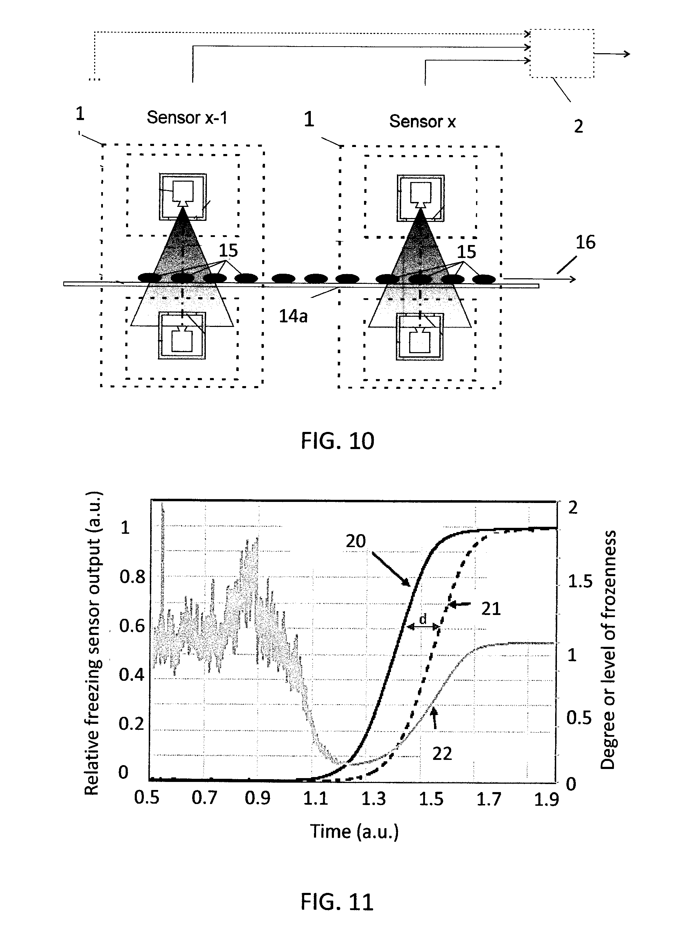

A monitoring system according to an embodiments of the present invention is shown in FIG. 10. It presents a configuration with two or more sensors 1 arranged on a freezing line such that each of the sensors measures products with a different process residence time. A substantially transparent conveyor belt 14a moves the products according to the arrow 16. Hence, sensor x measures products with a longer residence time in the freezer as compared to sensor x-1. A data processing unit 2 extracts the degree of frozenness out of both signals. The data processing algorithm may include (but is not limited thereto) the calculation of the absolute mathematical difference of sensor signals, the calculation of the ratio of sensor signals, a combination of more advanced data processing techniques such as curve-fitting, filtering, statistical analysis, etc. This configuration can be extended to a large series of sensor devices whereby the products 15 are measured at multiple product residence times in the freezer in order to capture the dynamic evolution of the freezing process. The "residence time difference" d is a function of the physical spacing between the sensor devices and the product speed defined by the product carrying medium 14.

The residence time difference d is shown in the graph of FIG. 11, in which the output of the sensor for long product residence LPR (given by the output of sensor x of FIG. 10) is indicated with a full line 20, and the output for short product residence SPR (output of sensor x-1 of FIG. 10) is indicated with a dashed line 21. The ratio R between the SPR and LPR is shown with a continuous line 22 (only with a meaningful value when the values of outputs are not zero).

The transceiver and/or receiver may comprise suitable electronics, such as power sources, signal conditioning and processing means and interface electronics for industrial networks, etc.

The sensors, measurement systems, methods and computer implementations according to embodiments of the present invention allow to optimize the industrial freezing process (e.g. freezing of food in alimentary industry, biotechnology, medicine, pharmacy, etc) in terms of product throughput and energy consumption with real-time expert knowledge about the degree of frozenness for the product under consideration. The present invention also allows to provide a good quality control in cold stores. It may allow optimization of temperature, wind speed, the timing of thawing of condensers in a freezing line, improve throughput, etc.

## Claims

1. A sensor system for sensing a level of frozenness of a predetermined product, the sensor system comprising
- at least one emission antenna for directing electromagnetic radiation with at least one frequency within a frequency range of 0.1 GHz to 10 THz to the product,
- at least one receiver for receiving said electromagnetic radiation after transmission through or reflection from said product,
- at least one data processing unit for determining a level of frozenness based on said received electromagnetic radiation and taking into account dielectric properties of the predetermined product based on a physical-mathematical model of the product or calibration data.

2. The sensor system according to claim 1, wherein the electromagnetic radiation is electromagnetic radiation of a single frequency or single frequency band.

3. The sensor system according to any of the previous claims, wherein the at least one emission antenna and the at least one receiver are configured with respect to each other and a product holder for holding the product so that the sensor system operates in transmission mode.

4. The sensor system according to any of the previous claims, wherein the data processing unit is adapted for taking into account a physical-mathematical model expressing a freezable water content being present in the predetermined product.

5. The sensor system according to any of the previous claims, wherein the data processing unit is programmed for taking into account the product thickness of the predetermined product for determining the fraction of frozen water of the freezable water content present in the predetermined product.

6. The sensor system according to any of the previous claims, wherein at least one of the emitter antenna or the receiver are separated from a region where the predetermined product is present, through a protective window.

7. The sensor system according to claim 6, wherein the protective window is dimensioned to have anti-reflective properties or wherein the protective window comprises a dedicated anti-reflective coating.

8. The sensor system according to any of the previous claims, the sensor system furthermore comprising a product transporting means, wherein the emission antenna, the receiver and the product transporting means are configured for detecting a level of frozenness of the predetermined product at a particular position of the predetermined product on the product transporting means.

9. The sensor system according to claim 8, wherein the system comprises a plurality of receivers along the product transporting means for detecting the level of frozenness at different positions along the product transporting means.

10. The sensor system according to any of the previous claims, wherein the system comprises a fill factor detector for detecting a fill factor.

11. The sensor system according to claim 10, wherein the frozenness monitoring system is adapted for accepting or rejecting a measurement when the fill factor is sufficiently high and/or wherein the data processing unit is adapted for taking into account the fill factor when determining a level of frozenness.

12. A method of detecting a level of frozeness of a predetermined product, the method comprising the steps of
- emitting electromagnetic radiation towards the predetermined product, the electromagnetic radiation having at least one frequency within a frequency range of 0.1 GHz to 10 THz - detecting electromagnetic radiation transmitted through or reflected from the predetermined product, and
- calculating a level of frozenness of the predetermined product based on said received electromagnetic radiation and taking into account dielectric properties of the predetermined product based on a physical-mathematical model of the product or calibration data.

13. A computer program product programmed for receiving data regarding an amount of electromagnetic radiation with at least one frequency within a frequency range of 0.1 GHz to 10 THz directed to the product and regarding an amount of electromagnetic radiation received after transmission through or reflection from said product, the computer program product further being programmed for determining a level of frozenness based on said received electromagnetic radiation and taking into account dielectric properties of the predetermined product based on a physical-mathematical model of the product or calibration data.

14. A freezing or thawing system, the system comprising a sensor system according to any of claims 1 to 11, whereby the system furthermore is adapted with a controller for controlling the freezing or thawing of products taking into account a determined level of frozenness.

15. Use of a sensor system according to any of claims 1 to 11
- for controlling a freezing or thawing system, or
- for analyzing a freezing or thawing cycle that was implemented on a predetermined product.
